# EUROPEAN PATENT APPLICATION

(11) **EP 4 681 717 A1**
(43) Date of publication of application: **21.01.2026**
(21) Application number: 24770963.7
(22) Date of filing: 14.03.2024
(51) Int. Cl.: A61K 31/785, A61K 47/59, A61K 47/60, A61P 27/02

(54) **EYE DROPS**

(30) Priority: 16.03.2023 JP 2023042003
(71) Applicant: NOF Corporation, Shibuya-ku Tokyo 150-6019 (JP)
(72) Inventor: SUMI, Azusa, Kawasaki-shi, Kanagawa 210-0865 (JP); SASAKI, Yumiko, Kawasaki-shi, Kanagawa 210-0865 (JP); HARA, Masao, Kawasaki-shi, Kanagawa 210-0865 (JP)
(74) Representative: Müller-Boré & Partner Patentanwälte PartG mbB
(86) International application number: PCT/JP2024/009965
(87) International publication number: WO 2024/190860

(57) **Abstract**

A ophthalmic solution of the present invention contains: (A) 0.02 w/w% or more and 0.15 w/w% or less of a 2-methacryloyloxyethyl phosphorylcholine-dimethylacrylamide-stearyl methacrylate copolymer, in which a molar ratio of constitutional units represented by formula (1) is a:b:c=100:10 to 400:2 to 50; (B) 0.000001 w/w% or more and 0.0001 w/w% or less of polyhexamethylene biguanide represented by formula (2) or a salt thereof; and (C) 0.02 w/w% or more and 2.0 w/w% or less of polyoxyethylene polyoxypropylene glycol, in which a content of sodium chloride is 0.3 w/w% or less.

According to the present invention, an ophthalmic solution having sufficient preservative efficacy and less eye irritancy while imparting lubricating property to a corneal surface can be provided.

## Description

### Technical Field

The present invention relates to an ophthalmic solution.

### Background Art

In recent years, there has been a rapid increase in the number of patients with dry eye due to wearing a contact lens, working with a smartphone or a personal computer, and the like. Causes of such dry eye include an increase in the tear evaporation amount due to a decrease in the number of blinks, friction between the palpebral conjunctiva and the ocular surface that occurs at the time of blinking, and drying of the eye due to breath leaking from the mask. Therefore, an ophthalmic solution used for dry eye is required to have moisture-retaining property for suppressing evaporation of a tear and lubricating property for reducing friction at the time of blinking.

In general, since an ophthalmic solution is repeatedly used for several weeks after opening, a preservative is blended in order to prevent contamination of bacteria in the container. In this regard, it is important that the preservative used in the ophthalmic solution exhibits sufficient preservative efficacy, and for example, polyhexamethylene biguanide and the like are frequently used as the preservative.

In addition, a technique for improving lubricating property and moisture-retaining property by blending a polymer containing a phosphorylcholine group in an ophthalmic solution is known. For example, PTL 1 discloses an ophthalmic solution containing a polymer containing a phosphorylcholine group. By using the ophthalmic solution described in PTL 1, the lubricating property of the corneal surface is improved, and the friction between the palpebral conjunctiva and the ocular surface at the time of blinking is reduced. Further, PTL 2 discloses an ophthalmic solution containing a polymer containing a phosphorylcholine group and polyhexamethylene biguanide. By using the ophthalmic solution described in PTL 2, it has sufficient preservative efficacy, quickly spreads on the eye or the surface of the contact lens, and exhibits moisturizing and lubricating effects.

### Citation List

### Patent Literature

PTL 1: WO2016/140242
PTL 2: JP2021-20856A

### Non Patent Literature

NPL 1: New Ophthalmology, Vol. 28, No. 3, Kazuyuki Isenoumi et al., Medical Aoi Publications, Inc., issued on March 31, 2011, pages 421 to 424

### Summary of Invention

### Technical Problem

By the way, in PTL 1, it is reported that sufficient lubricating property was imparted by blending a polymer containing a phosphorylcholine group in an ophthalmic solution, but the preservative efficacy is not clearly described. PTL 2 discloses a method of imparting the lubricating property and moisture-retaining property and exhibiting sufficient preservative efficacy with an ophthalmic solution in which a polymer containing a phosphorylcholine group, polyhexamethylene biguanide, and polyoxyethylene polyoxypropylene glycol are blended. However, the case where only a sufficient amount of a ternary copolymer which greatly contributes to improvement in lubricating property is blended is not clearly described, and there is also no description about sodium chloride. When a sufficient amount of the specific ternary copolymer described in PTLs 1 and 2, which greatly contributes to the improvement of lubricating property, is blended in order to improve the moisture-retaining property and lubricating property of the ophthalmic solution and polyhexamethylene biguanide is used as a preservative, there is a case where sufficient preservative efficacy is not obtained.

In addition, although polyhexamethylene biguanide has sufficient preservative efficacy as described above, when a contact lens washed with a multi-purpose solution in which polyhexamethylene biguanide is blended is worn on a cornea which is damaged or in which the amount of tears is reduced due to dry eye and the barrier function is reduced, there is a possibility that corneal disorder or corneal infection is caused (see, for example, NPL 1). Specifically, in the case of a cornea with a reduced barrier function or a damaged cornea, not only a multi-purpose solution, but also even an ophthalmic solution in which a preservative having high preservative efficacy is blended may cause irritating sensation in some patients in some cases. Therefore, there is a need for an ophthalmic solution with low eye irritancy that can be instilled even onto a cornea with reduced barrier function or a damaged cornea.

That is, for the treatment of dry eye, an ophthalmic solution having high lubricating property, sufficient preservative efficacy, and low eye irritancy is required.

The present invention has been made in view of the above problems, and an object is to provide an ophthalmic solution having high lubricating property and preservative efficacy and can reduce eye irritancy.

### Solution to Problem

As a result of intensive studies, the present inventors have found that an ophthalmic solution containing a 2-methacryloyloxyethyl phosphorylcholine-dimethylacrylamide-stearyl methacrylate copolymer (hereinafter, also referred to as a "copolymer (P)"), which is a ternary copolymer containing a phosphorylcholine group, a specific preservative, and a specific surfactant, at a specific ratio has high lubricating property and preservative efficacy and can reduce the eye irritancy, based on the new finding that sodium chloride, which is one of the constituent components of tear blended in an ophthalmic solution, reduces the preservative activity of polyhexamethylene biguanide, thereby completing the present invention.

The present invention according to the above findings is as follows.
[1] An ophthalmic solution containing: 0.02 w/w% or more and 0.15 w/w% or less of a 2-methacryloyloxyethyl phosphorylcholine-dimethylacrylamide-stearyl methacrylate copolymer, in which a molar ratio of constitutional units represented by formula (1) is a:b:c=100:10 to 400:2 to 50; 0.000001 w/w% or more and 0.0001 w/w% or less of polyhexamethylene biguanide represented by formula (2) or a salt thereof; and 0.02 w/w% or more and 2.0 w/w% or less of polyoxyethylene polyoxypropylene glycol, in which a content of sodium chloride is 0.3 w/w% or less. 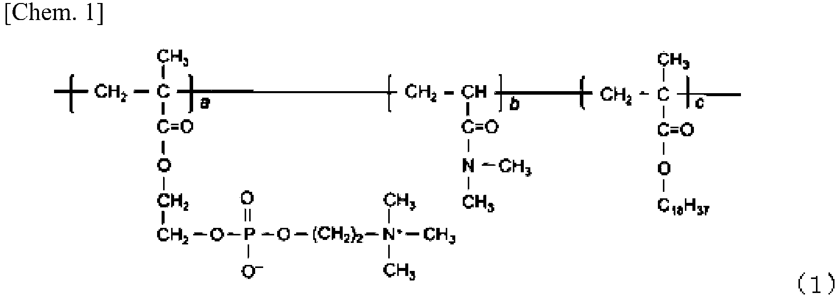
[In the formula (1), a, b, and c each represent a molar ratio of each constitutional unit.] 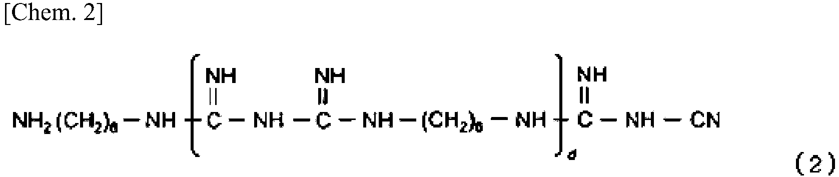 [In the formula (2), d is a number representing a repeating unit and is an integer of 3 or more and 40 or less.]

### Advantageous Effects of Invention

The ophthalmic solution of the present invention contains a 2-methacryloyloxyethylphosphorylcholine-dimethylacrylamide-stearyl methacrylate copolymer which is a ternary copolymer containing a phosphorylcholine group, polyhexamethylene biguanide, and polyoxyethylene polyoxypropylene glycol, and has a content of sodium chloride of 0.3 w/w% or less. Therefore, the ophthalmic solution can exhibit sufficient preservative efficacy and reduce the eye irritancy while imparting the lubricating property to a cornea. That is, according to the present invention, an ophthalmic solution having excellent lubricating property and sufficient preservative efficacy and having low eye irritancy can be provided.

### Description of Embodiments

The ophthalmic solution of the present invention contains: 0.02 w/w% or more and 0.15 w/w% or less of a 2-methacryloyloxyethyl phosphorylcholine-dimethylacrylamide-stearyl methacrylate copolymer, in which a molar ratio of constitutional units represented by formula (1) is a:b:c=100:10 to 400:2 to 50, as a component (A); 0.000001 w/w% or more and 0.0001 w/w% or less of polyhexamethylene biguanide represented by formula (2) or a salt thereof, as a component (B); and 0.02 w/w% or more and 2.0 w/w% or less of polyoxyethylene polyoxypropylene glycol, as a component (C), in which a content of sodium chloride is 0.3 w/w% or less. 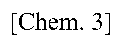 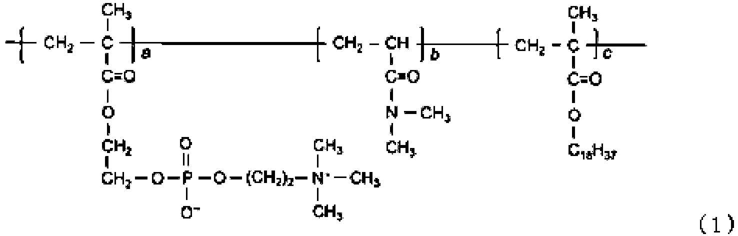 [In the formula (1), a, b, and c each represent a molar ratio of each constitutional unit.] 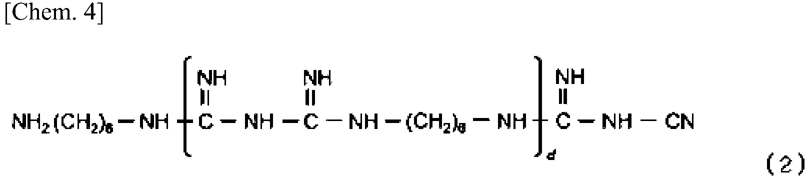 [In the formula (2), d is a number representing a repeating unit and is an integer of 3 or more and 40 or less.]

The component (A) used in the ophthalmic solution of the present invention has three constitutional units of 2-methacryloyloxyethyl phosphorylcholine, dimethylacrylamide, and stearyl methacrylate, represented by the above formula (1), and the molar ratio of each constitutional unit a:b:c is 100:10 to 400:2 to 50. When the ophthalmic solution contains the component (A), the lubricating property is improved and the eye irritancy is reduced.

When the number of moles a of 2-methacryloyloxyethyl phosphorylcholine is 100, the number of moles b of dimethylacrylamide is b/a=10/100 or more and 400/100 or less, and preferably 30/100 or more and 250/100 or less. When the number of moles of dimethylacrylamide is too large, there is a possibility that aseptic filtration necessary for producing an ophthalmic solution is difficult, and when it is too small, an effect of the improving lubricating property cannot be expected.

When the number of moles a of 2-methacryloyloxyethyl phosphorylcholine is 100, the number of moles c of stearyl methacrylate is c/a=2/100 or more and 50/100 or less, and preferably 5/100 or more and 25/100 or less. When the number of moles of stearyl methacrylate is too large, the hydrophilicity of the copolymer (P) is reduced so that the solubility in water is reduced, and it becomes difficult to produce an ophthalmic solution. When the number of moles of stearyl methacrylate is too small, the effect of the improving lubricating property is not sufficiently maintained.

Further, the weight average molecular weight of the copolymer (P) is 5,000 to 2,000,000. When the weight average molecular weight is less than 5,000, the polymer does not have sufficient adsorptivity to the surface of the contact lens, so that there is a possibility that improvement in the lubricating property is not expected, and when it is more than 2,000,000, there is a possibility that aseptic filtration necessary for producing an ophthalmic solution is difficult. Further, from the viewpoint of improving the lubricating property, the weight average molecular weight of the copolymer (P) is preferably 100,000 to 1,500,000. Note that the weight average molecular weight means a value in terms of polyethylene glycol (PEG) measured by gel permeation chromatography (GPC).

The copolymer (P) represented by the formula (1) has the respective constitutional units derived from 2-methacryloyloxyethyl phosphorylcholine, dimethylacrylamide, and stearyl methacrylate, but the arrangement of the constitutional units in the copolymer (P) is not particularly limited, and can be a random or a block.

The copolymer (P) of the component (A) can be produced, for example, as follows. It can be produced by radical polymerization of 2-methacryloyloxyethyl phosphorylcholine, dimethylacrylamide, and stearyl methacrylate in the presence of a radical polymerization initiator and under substitution or atmosphere of an inert gas such as nitrogen, carbon dioxide, argon, or helium. The polymerization method can be performed by a known method such as bulk polymerization, suspension polymerization, emulsion polymerization, or solution polymerization. Purification of the copolymer (P) can be performed by a known method such as a reprecipitation method, a dialysis method, or an ultrafiltration method.

The blending amount of the component (A) in the ophthalmic solution of the present invention is 0.02 w/w% or more and 0.15 w/w% or less. When it is less than 0.02 w/w%, there is a possibility that the lubricating property is not sufficiently exhibited. When it is more than 0.15 w/w%, there is a possibility that the preservative efficacy by the preservative is inhibited. From the viewpoint of exhibiting the preservative efficacy of the preservative and the lubricating property, the blending amount of the component (A) is preferably 0.075 w/w% or more and 0.125 w/w% or less.

The component (B) is a compound represented by the above formula (2), and is referred to as polyhexamethylene biguanide. In addition, it can also be a salt thereof. In the above formula (2), d is a number representing a repeating unit and is an integer of 3 or more and 40 or less. When the value of d is less than 3 or more than 40, there is a possibility that the preservative efficacy as an ophthalmic solution is not sufficiently exhibited. From the viewpoint of exhibiting more sufficient preservative efficacy, the value of d is preferably 10 or more and 18 or less.

Examples of the salt of polyhexamethylene biguanide include a hydrochloride, a borate, an acetate, a gluconate, a sulfonate, a tartrate, and a citrate. Specific examples thereof include a commercially available product such as Cosmocil CQ (registered trademark) and Vantcil IB (registered trademark), manufactured by Arch UK Biocides, and BG-1 manufactured by Sanyo Chemical Industries, Ltd. One of these can be selected and used alone, or two or more of these can be used in any combination.

The blending amount of the component (B) in the ophthalmic solution of the present invention is 0.000001 w/w% or more and 0.0001 w/w% or less. When it is less than 0.000001 w/w%, there is a possibility that the preservative efficacy is not sufficiently exhibited as an ophthalmic solution. When it is more than 0.0001 w/w%, there is a possibility that the improvement of the preservative efficacy commensurate with the blending amount is not expected, and the cell survival rate decreases. The blending amount of the polyhexamethylene biguanide or a salt thereof is preferably 0.00001 w/w% or more and 0.00002 w/w% or less from the viewpoint of improving the preservative efficacy commensurately with the blending amount and not decreasing the cell survival rate.

The polyoxyethylene polyoxypropylene glycol of the component (C) can be one used in an ordinary pharmaceutical product, and for example, a product listed in the Japanese Pharmacopoeia can be used. Specific examples thereof include a commercially available product such as UNILUBE (registered trademark) 70DP 950B manufactured by NOF Corporation, Japanese Pharmaceutical Excipient PLONON (registered trademark) 188P manufactured by NOF Corporation, and NEWPOL (registered trademark) PE-68 manufactured by Sanyo Chemical Industries, Ltd.

The blending amount of the component (C) in the ophthalmic solution of the present invention is 0.02 w/w% or more and 2.0 w/w% or less. When the blending amount of the component (C) is less than 0.02 w/w%, there is a possibility that the preservative efficacy decreases, and when it is more than 2.0 w/w%, there is a possibility that the preservative efficacy decreases and the cell survival rate decreases. The blending amount of polyoxyethylene polyoxypropylene glycol is preferably 0.1 w/w% or more and 1.0 w/w% or less, from the viewpoint of not decreasing the preservative efficacy and not decreasing the cell survival rate.

The content of sodium chloride in the ophthalmic solution of the present invention is 0.3 w/w% or less. When the content of sodium chloride is more than 0.3 w/w%, there is a possibility that the preservative efficacy decreases. Note that, it is not necessary to contain sodium chloride in the ophthalmic solution of the present invention, and when the content of sodium chloride is 0.1 w/w% or more and 0.3 w/w% or less, the eye irritancy is further reduced.

The ophthalmic solution of the present invention can contain components other than the above components (A) to (C). Examples of the other components can include a polyhydric alcohol, an algefacient, an inorganic salt, a salt of an organic acid, an acid, a base, and an antioxidant.

Examples of the polyhydric alcohol include propylene glycol, glycerin, glucose, mannitol, sorbitol, xylitol, and trehalose.

Examples of the algefacient include menthol and camphor.

Examples of the inorganic salt include potassium chloride, borax, sodium hydrogen carbonate, sodium hydrogen phosphate, and anhydrous sodium dihydrogen phosphate.

Examples of the salt of organic acid include sodium citrate and sodium acetate.

Examples of the acid include boric acid, phosphoric acid, citric acid, sulfuric acid, acetic acid, and hydrochloric acid.

Examples of the base include sodium hydroxide, potassium hydroxide, trometamol, and monoethanolamine.

Examples of the antioxidant include tocopherol acetate and dibutylhydroxytoluene. Examples

Hereinafter, the present invention is specifically described with reference to Examples, but the present invention is not limited to the scope of the following Examples.

The copolymers and homopolymers used in the following Examples and Comparative Examples are as follows.

### Copolymer (P)

Polymer 1: Copolymer of 2-methacryloyloxyethyl phosphorylcholine (MPC), dimethylacrylamide, and stearyl methacrylate (copolymerization composition ratio of MPC, dimethylacrylamide, and stearyl methacrylate [MPC/dimethylacrylamide/stearyl methacrylate (molar ratio) =10/9/1], weight average molecular weight: 100,000)
Polymer 2: Copolymer of 2-methacryloyloxyethyl phosphorylcholine (MPC), dimethylacrylamide, and stearyl methacrylate (copolymer composition ratio of MPC, dimethylacrylamide, and stearyl methacrylate [MPC/dimethylacrylamide/stearyl methacrylate (molar ratio) =5/4/1], weight average molecular weight: 100,000)
Copolymer (Q): Copolymer of 2-methacryloyloxyethyl phosphorylcholine (MPC) and butyl methacrylate (copolymer composition ratio of MPC and butyl methacrylate [MPC/butyl methacrylate (molar ratio) =4/1], weight average molecular weight: 600,000)

### [Preparation of Ophthalmic Solution]

### (Example 1)

About 50 g of purified water was heated to 45°C, to which 0.1 g of potassium chloride, 0.4 g of boric acid, 0.04 g of borax, 0.00008 g of Cosmocil CQ (registered trademark) (because it is a 20% aqueous solution, 0.000016 g is contained as polyhexanide hydrochloride [polyhexamethylene biguanide hydrochloride]) as the component (B), 1.8 g of concentrated glycerin, 0.2 g of UNILUBE 70DP-950B as the component (C), and 10 g of the copolymer (P) (because it is a 1 % aqueous solution, 0.1 g is contained as a 2-methacryloyloxyethyl phosphorylcholine-dimethylacrylamide-stearyl methacrylate copolymer) as the component (A) were added sequentially and stirred. Thereafter, purified water was added thereto such that the total amount was 100 g. Then, the filter sterilization was performed to prepare an aseptic ophthalmic solution.

Note that, the Cosmocil CQ (registered trademark) is a compound represented by the formula (2) in which d=14.

### (Examples 2 to 10)

Each ophthalmic solution was prepared in the same manner as in Example 1 using the kinds and amounts of components shown in Table 1.

### (Comparative Examples 1 to 11)

Each ophthalmic solution was prepared in the same manner as in Example 1 using the kinds and amounts of components shown in Table 2. Note that as the hyaluronic acid, BIO-SODIUM HYALURONATE POWDER (MMW) powder (manufactured by HYUNDAI BIOLAND) was used.

### [Table 1]

**Table 1**

| Blended Component (w/w%) | | Example 1 | Example 2 | Example 3 | Example 4 | Example 5 |
|---|---|---|---|---|---|---|
| Potassium Chloride | | 0.10 | 0.10 | 0.10 | 0.10 | 0.10 |
| Sodium Chloride | | | | | | 0.30 |
| Copolymer (P) | Polymer 1 | 0.10 | | 0.10 | 0.10 | 0.10 |
| | Polymer 2 | | 0.10 | | | |
| Boric Acid | | 0.40 | 0.40 | 0.40 | 0.40 | 0.40 |
| Borax | | 0.04 | 0.04 | 0.04 | 0.04 | 0.04 |
| Polyhexanide Hydrochloride | | 0.000016 | 0.000016 | 0.000016 | 0.0001 | 0.000016 |
| Concentrated Glycerin | | 1.80 | 1.80 | 1.80 | 1.80 | 1.60 |
| UNILUBE 70DP-950B | | 0.20 | 0.20 | 0.02 | 0.20 | 0.20 |
| Purified Water | | Remaining Parts | Remaining Parts | Remaining Parts | Remaining Parts | Remaining Parts |

**Table 1 (continued)**

| Blended Component (w/w%) | | Example 6 | Example 7 | Example 8 | Example 9 | Example 10 |
|---|---|---|---|---|---|---|
| Potassium Chloride | | 0.10 | 0.10 | 0.10 | 0.10 | 0.10 |
| Sodium Chloride | | | | | | |
| Copolymer (P) | Polymer 1 | 0.10 | 0.15 | 0.02 | 0.10 | 0.02 |
| | Polymer 2 | | | | | |
| Boric Acid | | 0.40 | 0.40 | 0.40 | 0.40 | 0.40 |
| Borax | | 0.04 | 0.04 | 0.04 | 0.04 | 0.04 |
| Polyhexanide Hydrochloride | | 0.000001 | 0.000016 | 0.000016 | 0.000016 | 0.000001 |
| Concentrated Glycerin | | 1.80 | 1.80 | 1.80 | 1.80 | 1.80 |
| UNILUBE 70DP-950B | | 0.20 | 0.20 | 0.20 | 2.00 | 0.20 |
| Purified Water | | Remaining Parts | Remaining Parts | Remaining Parts | Remaining Parts | Remaining Parts |

### [Table 2]

**Table 2**

| Blended Component (w/w%) | Comparative Example 1 | Comparative Example 2 | Comparative Example 3 | Comparative Example 4 | Comparative Example 5 | Comparative Example 6 |
|---|---|---|---|---|---|---|
| Potassium Chloride | 0.10 | 0.10 | 0.10 | 0.10 | 0.10 | 0.10 |
| Sodium Chloride | | | 0.60 | | | |
| Copolymer (P) Polymer 1 | 0.10 | 0.10 | 0.10 | 0.01 | 0.50 | 0.10 |
| Copolymer (Q) | | | | 0.09 | | |
| Hyaluronic Acid | | | | | | |
| Boric Acid | 0.40 | 0.40 | 0.40 | 0.40 | 0.40 | 0.40 |
| Borax | 0.04 | 0.04 | 0.04 | 0.04 | 0.04 | 0.04 |
| Polyhexanide Hydrochloride | 0.000016 | 0.001 | 0.000016 | 0.000016 | 0.000016 | |
| Concentrated Glycerin | 1.80 | 1.80 | | 1.80 | 1.80 | 1.80 |
| UNILUBE 70DP-950B | | 0.20 | 0.20 | 0.20 | 0.20 | 0.20 |
| Purified Water | Remaining Parts | Remaining Parts | Remaining Parts | Remaining Parts | Remaining Parts | Remaining Parts |

**Table 2 (continued)**

| Blended Component (w/w%) | Comparative Example 7 | Comparative Example 8 | Comparative Example 9 | Comparative Example 10 | Comparative Example 11 |
|---|---|---|---|---|---|
| Potassium Chloride | 0.10 | 0.10 | 0.10 | 0.10 | 0.10 |
| Sodium Chloride | | | | | |
| Copolymer (P) Polymer 1 | 0.01 | | | 0.10 | 0.01 |
| Copolymer (Q) | | | | | |
| Hyaluronic Acid | | 0.10 | | | |
| Boric Acid | 0.40 | 0.40 | 0.40 | 0.40 | 0.40 |
| Borax | 0.04 | 0.04 | 0.04 | 0.04 | 0.04 |
| Polyhexanide Hvdrochloride | 0.000016 | 0.000016 | 0.000016 | 0.000016 | 0.00000015 |
| Concentrated Glycerin | 1.80 | 1.80 | 1.80 | 1.80 | 1.80 |
| UNILUBE 70DP-950B | 0.20 | | 0.20 | 5.00 | 10.00 |
| Purified Water | Remaining Parts | Remaining Parts | Remaining Parts | Remaining Parts | Remaining Parts |

### [Evaluation]

The above Examples and Comparative Examples were evaluated as follows.

### (1) Evaluation of Lubricating Property

A gel plate immersed in the ophthalmic solution of each of Examples 1 to 10 and Comparative Examples 1 to 11 was evaluated for slipperiness when rubbed with a finger by sensory evaluation.

### (Preparation of Gel Plate)

(1) To a brown screw tube, 19.8166 g of 2-hydroxyethyl methacrylate (HEMA) (manufactured by Mitsubishi Chemical Group Corporation), 0.2011 g of methacrylic acid (MAA) (manufactured by Tokyo Chemical Industry Co., Ltd.), 0.1429 g of ethylene glycol dimethacrylate (manufactured by Tokyo Chemical Industry Co., Ltd.), and 0.1005 g of 2,2-azobis (isobutyronitrile) (manufactured by FUJIFILM Wako Pure Chemical Corporation) were added and mixed by stirring with a wave rotor for 1 hour.
(2) Dispensing into cells for the plate material preparation was performed with a pipetter.
(3) Curing was performed under a condition at 100°C for 1 hour.
(4) The cured gel was immersed in ISO saline to obtain HEMA-MAA gel.

The gel plate was immersed in about 50 mL of the ophthalmic solution of each of Examples 1 to 10 and Comparative Examples 1 to 11, then the gel plate was taken out, and the slipperiness when rubbed with a finger was evaluated according to the following evaluation criteria. The average score of the evaluation scores of the seven panelists was calculated, and the lubricating property was evaluated according to the following criteria for determination.

### (Evaluation Criteria)

Good slipperiness of gel plate: 3 points
Slightly good slipperiness of gel plate: 2 points
Slightly poor slipperiness of gel plate: 1 point
Poor slipperiness of gel plate: 0 point

### (Criteria for Determination)

A case where the average score of the evaluation scores was 2 points or more and less than 3 points was evaluated as good "A", a case where it was 1.5 points or more and less than 2 points was evaluated as fair "B", and a case where it was 0 point or more and less than 1.5 points was evaluated as poor "C".

The test results are shown in Table 3. In Examples 1 to 10 in which the copolymer (P) was blended, the slipperiness of the gel plate was better compared to Comparative Example 9 in which the copolymer (P) was not blended. As described above, the ophthalmic solution of the present invention exhibits excellent lubricating property.

### [Table 3]

**Table 3**

| | Example 1 | Example 2 | Example 3 | Example 4 | Example 5 |
|---|---|---|---|---|---|
| Evaluation Score (Score) | 2.4 | 2.3 | 2.1 | 2.3 | 2.3 |
| Determination | A | A | A | A | A |

**Table 3 (continued)**

| | Example 6 | Example 7 | Example 8 | Example 9 | Example 10 |
|---|---|---|---|---|---|
| Evaluation Score (Score) | 2.2 | 2.8 | 1.8 | 2.5 | 1.6 |
| Determination | A | A | B | A | B |

**Table 3 (continued)**

| | Comparative Example 1 | Comparative Example 2 | Comparative Example 3 | Comparative Example 4 | Comparative Example 5 | Comparative Example 6 |
|---|---|---|---|---|---|---|
| Evaluation Score (Score) | 2.0 | 2.3 | 2.4 | 1.4 | 3.0 | 2.4 |
| Determination | A | A | A | C | A | A |

**Table 3 (continued)**

| | Comparative Example 7 | Comparative Example 8 | Comparative Example 9 | Comparative Example 10 | Comparative Example 11 |
|---|---|---|---|---|---|
| Evaluation Score (Score) | 0.9 | 2.6 | 1.2 | 2.7 | 1.3 |
| Determination | C | A | C | A | C |

### (2) Preservative-Effectiveness Test

The preservative-effectiveness test was performed in accordance with the section of "Japanese Pharmacopoeia 18th Edition, General Information, Preservative-Effectiveness Test Method". Three species of Escherichia coli (E. coli, hereinafter abbreviated as E. c.) (NBRC3972), Pseudomonas aeruginosa (P. aeruginosa, hereinafter abbreviated as P. a.) (NBRC13275), and Staphylococcus aureus (S. aureus, hereinafter abbreviated as S. a.) (NBRC13276) were used as the bacteria, and two species of Candida albicans (candida, hereinafter abbreviated as C. a.) (NBRC1594) and Aspergillus brasiliensis (black aspergillus, hereinafter abbreviated as A. b.) (NBRC9455) were used as the fungi.

BIOBALL (registered trademark) (manufactured by bioMérieux Japan Ltd.) of each of E. c., P. a, S. a., C. a., and A. b. described above was redissolved to prepare a test bacterial solution having 10⁸ CFU (Colony Forming Unit, the number of units capable of forming colonies) per 1 mL. This test bacterial solution was added to each of Examples 1 to 10 and Comparative Examples 1 to 11 so as to be 1 v/v%, and the number of inoculated bacteria was adjusted to be 10⁶ CFU per 1 mL, and stored at 25°C.

Sampling was performed 14 days and 28 days after the inoculation, and the number of viable bacteria was measured by culturing. A case where no colony was generated after 28 days was determined as good "A", a case where a colony was generated after 28 days but the number of bacteria after 28 days did not increase from the number of bacteria after 14 days was determined as fair "B", and a case where the number of bacteria after 28 days increased from the number of bacteria after 14 days was determined as poor "C".

In addition, the overall evaluation was determined as good "A" in a case where a colony was not generated in all the bacterial species after 28 days, as fair "B" in a case where a colony was generated in at least two bacterial species after 28 days but the number of bacteria was less than the number of bacteria after 14 days, and as poor "C" in a case where the number of bacteria after 28 days increased more than the number of bacteria after 14 days in at least one bacterial species.

The results are shown in Table 4. In Examples 1 to 10, all the bacteria and fungi satisfied the criteria for determination. As described above, the ophthalmic solution of the present invention has sufficient preservative efficacy.

### [Table 4]

**Table 4**

| | Determination | | | | | Overall Evaluation |
|---|---|---|---|---|---|---|
| | E.c. | P.a. | S.a. | C.a. | A.b. | |
| Example 1 | A | A | A | A | B | A |
| Example 2 | A | A | A | A | B | A |
| Example 3 | A | A | A | B | B | B |
| Example 4 | A | A | A | A | A | A |
| Example 5 | A | A | A | A | B | A |
| Example 6 | B | B | B | B | B | B |
| Example 7 | A | B | A | B | B | B |
| Example 8 | A | A | A | A | A | A |
| Example 9 | A | A | A | A | B | A |
| Example 10 | B | B | B | B | B | B |

| | Determination | | | | | Overall Evaluation |
|---|---|---|---|---|---|---|
| | E.c. | P.a. | S.a. | C.a. | A.b. | |
| Comparative Example 1 | B | C | A | A | C | C |
| Comparative Example 2 | A | A | A | A | A | A |
| Comparative Example 3 | A | C | A | A | C | C |
| Comparative Example 4 | A | A | A | A | A | A |
| Comparative Example 5 | C | C | C | C | C | C |
| Comparative Example 6 | C | C | C | C | C | C |
| Comparative Example 7 | A | A | A | A | A | A |
| Comparative Example 8 | A | A | A | A | A | A |
| Comparative Example 9 | A | A | A | A | A | A |
| Comparative Example 10 | B | C | C | B | B | C |
| Comparative Example 11 | C | C | C | C | C | C |

### (3) Evaluation of Cell Survival Rate

The evaluation of the cell survival rate was performed using rabbit corneal epithelial cells (SIRC cells). SIRC cells pre-cultured in Dulbecco's modified Eagle medium (DMEM) containing 10 v/v% FBS (fetal bovine serum) in advance were treated with trypsin to prepare a cell suspension of 1.0×10⁵ cells/mL. The cell suspension was seeded in a 96-well plate at 0.1 mL/well and cultured for 24 hours to allow the cells to adhere, and then each of Examples 1 to 10 and Comparative Examples 1 to 11 was added at 0.1 mL/well and further cultured for 24 hours. The medium was removed and a 50 µg/mL neutral red solution was added to allow the live cells to take up neutral red. After 3 hours, the cells were washed with PBS (phosphate buffer saline), neutral red was extracted with an aqueous solution of 1 v/v% of acetic acid/ 50 v/v% of ethanol, and the absorbance at 540 nm was measured. The absorbance when the sample solution was not added was set at 100% cell survival rate. The cell survival rate in the case where each of Examples 1 to 10 and Comparative Examples 1 to 11 was added was calculated, and the 50% inhibition concentration (IC₅₀) was calculated. When the IC₅₀ was 50% or more and less than 100%, it was determined as "A" indicating that the cell survival rate was high, when the IC₅₀ was 40% or more and less than 50%, it was determined as "B" indicating that the cell survival rate was medium, and when the IC₅₀ was more than 0% and less than 40%, it was determined as "C" indicating that the cell survival rate was low.

The results are shown in Table 5. As compared to Comparative Example 9 in which the copolymer (P) was not blended and Comparative Example 11 in which the blending amount of the copolymer (P) was small, the cell survival rate was higher in Examples 1 to 10 in which the copolymer (P) was blended. As described above, the ophthalmic solution of the present invention has less eye irritancy.

### [Table 5]

**Table 5**

| | Example 1 | Example 2 | Example 3 | Example 4 | Example 5 |
|---|---|---|---|---|---|
| IC₅₀(%) | 58 | 55 | 56 | 42 | 60 |
| Determination | A | A | A | B | A |

**Table 5 (continued)**

| | Example 6 | Example 7 | Example 8 | Example 9 | Example 10 |
|---|---|---|---|---|---|
| IC₅₀(%) | 61 | 64 | 45 | 46 | 48 |
| Determination | A | A | B | B | B |

**Table 5 (continued)**

| | Comparative Example 1 | Comparative Example 2 | Comparative Example 3 | Comparative Example 4 | Comparative Example 5 | Comparative Example 6 |
|---|---|---|---|---|---|---|
| IC₅₀(%) | 59 | 39 | 59 | 50 | 68 | 61 |
| Determination | A | C | A | A | A | A |

**Table 5 (continued)**

| | Comparative Example 7 | Comparative Example 8 | Comparative Example 9 | Comparative Example 10 | Comparative Example 11 |
|---|---|---|---|---|---|
| IC₅₀(%) | 43 | 39 | 30 | 36 | 34 |
| Determination | B | C | C | C | C |

### (4) Evaluation of Cell Survival Rate after Instillation

In order to evaluate the cell survival rate after instillation, the state after instillation in which the ophthalmic solution of the present invention was mixed with tear was simulated, and the cell survival rate was evaluated using a human multi-layered corneal epithelium model (corneal model) (manufactured by Japan Tissue Engineering Co., Ltd.) for sample solutions obtained by adding 0.55 g of sodium chloride to the ophthalmic solution of each of Examples 1 to 10 and Comparative Examples 1 to 11. 0.1 mL of sample solution was added to each corneal model cultured in an assay medium (manufactured by Japan Tissue Engineering Co., Ltd.) in advance, and cultivation was performed for 24 hours. Thereafter, a reaction liquid of LDH Cytotoxicity Detection kit (manufactured by Takara Bio Inc.) was added. After 30 minutes, a reaction termination liquid was added, and the absorbance at 490 nm was measured. The LDH activity value, which is an index of the number of damaged cells, was calculated from the absorbance, and the cell survival rate after instillation was evaluated. When the LDH activity value was less than 500 Unit/L, the cell survival rate after instillation was rated as high "A", when the LDH activity value was 500 or more and less than 1,000 Unit/L, the cell survival rate after instillation was rated as medium "B", and when the LDH activity value was 1,000 Unit/L or more, the cell survival rate after instillation was rated as low "C".

The results are shown in Table 6. In Examples 1 to 10, the LDH activity value was low. In addition, the result of Example 1 was comparable to that of Comparative Example 6 in which polyhexanide hydrochloride was not contained. As described above, in the ophthalmic solution of the present invention, the eye irritancy is further reduced by mixing with the tear.

### [Table 6]

**Table 6**

| | Example 1 | Example 2 | Example 3 | Example 4 | Example 5 |
|---|---|---|---|---|---|
| LDH Activity Value (Unit/L) | 320 | 360 | 330 | 490 | 310 |
| Determination | A | A | A | A | A |

**Table 6 (continued)**

| | Example 6 | Example 7 | Example 8 | Example 9 | Example 10 |
|---|---|---|---|---|---|
| LDH Activity Value (Unit/L) | 300 | 290 | 780 | 650 | 550 |
| Determination | A | A | B | B | B |

**Table 6 (continued)**

| | Comparative Example 1 | Comparative Example 2 | Comparative Example 3 | Comparative Example 4 | Comparative Example 5 | Comparative Example 6 |
|---|---|---|---|---|---|---|
| LDH Activity Value (Unit/L) | 320 | 495 | 450 | 380 | 250 | 300 |
| Determination | A | A | A | A | A | A |

**Table 6 (continued)**

| | Comparative Example 7 | Comparative Example 8 | Comparative Example 9 | Comparative Example 10 | Comparative Example 11 |
|---|---|---|---|---|---|
| LDH Activity Value (Unit/L) | 890 | 1200 | 1300 | 1100 | 950 |
| Determination | B | C | C | C | B |

The results of all the test items are shown in Table 7. From the above results, it was found that the ophthalmic solution of the present invention has sufficient preservative efficacy and less eye irritancy while imparting the lubricating property to the corneal surface.

### [Table 7]

**Table 7**

| Test Item | Example 1 | Example 2 | Example 3 | Example 4 | Example 5 |
|---|---|---|---|---|---|
| Lubricating Property Test | A | A | A | A | A |
| Preservative-Effectiveness Test | A | A | B | A | A |
| Evaluation of Cell Survival Rate | A | A | A | B | A |
| Cell Survival Rate after Instillation | A | A | A | A | A |

**Table 7 (continued)**

| Test Item | Example 6 | Example 7 | Example 8 | Example 9 | Example 10 |
|---|---|---|---|---|---|
| Lubricating Property Test | A | A | B | A | B |
| Preservative-Effectiveness Test | B | B | A | A | B |
| Evaluation of Cell Survival Rate | A | A | B | B | B |
| Cell Survival Rate after Instillation | A | A | B | B | B |

**Table 7 (continued)**

| Test Item | Comparative Example 1 | Comparative Example 2 | Comparative Example 3 | Comparative Example 4 | Comparative Example 5 | Comparative Example 6 |
|---|---|---|---|---|---|---|
| Lubricating Property Test | A | A | A | C | A | A |
| Preservative-Effectiveness Test | C | A | C | A | C | C |
| Evaluation of Cell Survival Rate | A | C | A | A | A | A |
| Cell Survival Rate after Instillation | A | A | A | A | A | A |

**Table 7 (continued)**

| Test Item | Comparative Example 7 | Comparative Example 8 | Comparative Example 9 | Comparative Example 10 | Comparative Example 11 |
|---|---|---|---|---|---|
| Lubricating Property Test | C | A | C | A | C |
| Preservative-Effectiveness Test | A | A | A | C | C |
| Evaluation of Cell Survival Rate | B | C | C | C | C |
| Cell Survival Rate after Instillation | B | C | C | C | B |

### Industrial Applicability

According to the present invention, the ophthalmic solution having sufficient preservative efficacy and less eye irritancy while imparting the lubricating property to the corneal surface can be obtained.

## Claims

1. An ophthalmic solution comprising:
(A) 0.02 w/w% or more and 0.15 w/w% or less of a 2-methacryloyloxyethyl phosphorylcholine-dimethylacrylamide-stearyl methacrylate copolymer, in which a molar ratio of constitutional units represented by formula (1) is a:b:c=100:10 to 400:2 to 50;
(B) 0.000001 w/w% or more and 0.0001 w/w% or less of polyhexamethylene biguanide represented by formula (2) or a salt thereof; and
(C) 0.02 w/w% or more and 2.0 w/w% or less of polyoxyethylene polyoxypropylene glycol, wherein a content of sodium chloride is 0.3 w/w% or less: 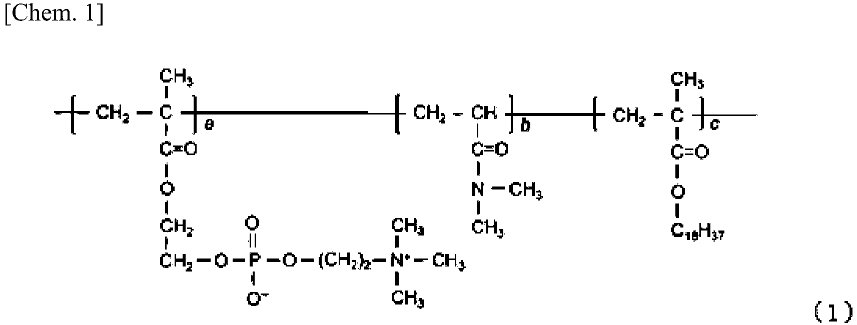 wherein, in the formula (1), a, b, and c each represent a molar ratio of each constitutional unit; and 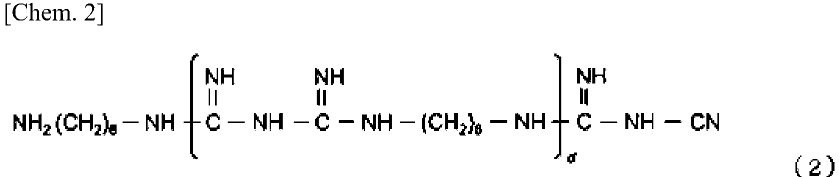 wherein, in the formula (2), d is a number representing a repeating unit and is an integer of 3 or more and 40 or less.
